# EUROPÄISCHE PATENTSCHRIFT

(11) **EP 0 653 197 B1**
(45) Veröffentlichungstag und Bekanntmachung des Hinweises auf die Patenterteilung: **02.06.1999**
(21) Anmeldenummer: 93118417.0
(22) Anmeldetag: 15.11.1993
(51) Int. Cl.: A61F 9/00, A61B 17/02

(54) **Operationshaken zur Verwendung beim Eingriff am Auge eines Lebewesens**
Hook for use in eye surgery
Crochet à utiliser dans la chirurgie de l'oeuil

(43) Veröffentlichungstag der Anmeldung: 17.05.1995
(73) Patentinhaber: DUKE UNIVERSITY MEDICAL CENTER, Durham, North Carolina 27710 (US); GRIESHABER & CO. AG SCHAFFHAUSEN, CH-8203 Schaffhausen (CH)
(72) Erfinder: Hickingbotham, Dyson, Bahama, NC 27503 (US); De Juan, Eugene, Jr., Durham, NC 27707 (US)
(74) Vertreter: Althoff, Gerhard

(56) Entgegenhaltungen:
- EP-A- 0 502 258
- DE-U- 8 712 620
- US-A- 4 037 589
- US-A- 4 239 036
- SOVIET INVENTIONS ILLUSTRATED Section PQ, Week 8346, 28. Dezember 1983 Derwent Publications Ltd., London, GB; Class P32, AN 83-819217 & SU-A-990 220 (MOSC EYE MICROSURGERY) 28. Januar 1983

## Beschreibung

Die Erfindung bezieht sich auf einen Operationshaken zur Verwendung beim chirurgischen Eingriff am Auge eines Lebewesens, bestehend aus einem zum örtlichen Zurückziehen der Regenbogenhaut an mindestens einem Ende mit einem angeformten hakenförmigen Einhängeteil sowie mit einem länglichen Führungsteil versehenen Hakenelement aus einem flexiblen Kunststoffaden und einem am Führungsteil verschiebbar angeordneten Fixierelement, mit welchem der durch einen Einschnitt in der Hornhaut eingeführte Operationshaken an der äusseren Kontur des Auges haltbar ist.

Bei der Augenchirurgie besteht vielfach das Problem, dass die Patienten auf medikamentöse Vergrösserung der Iris nicht ansprechen oder die Vergrösserung der Pupillenöffnung für den operativen Eingriff nicht ausreichend ist. Insbesondere bei Katarakt-Operationen ist es für den operativen Eingriff im vorderen Augenabschnitt aber auch bei Eingriffen im hinteren Augenabschnitt erforderlich ein ausreichend grosses, während des operativen Eingriffs unverändert bestehenbleibendes Sichtfeld für den Operateur zu gewährleisten. Zur Bildung des entsprechenden Sichtfeldes ist es bekannt, mittels einem oder mehrerer im Abstand zueinander angeordneter Operationshaken (Iris-Retractor) die Regenbogenhaut (Iris) zu erfassen und zur Vergrösserung der Pupillenöffnung nach aussen zu ziehen. Die einzelnen Operationshaken werden dabei jeweils durch einen entsprechend in der Hornhaut (Cornea) vorgesehenen Einschnitt eingeführt, am Auge positioniert und nach dem Eingriff wieder entfernt.

Für operative Eingriffe am Auge eines Lebewesens ist aus der US-A 4,037,589 ein als Drahtgestell ausgebildetes Rückhaltesystem mit einem ersten, in einem Gleitstück verstellbar angeordneten Operationshaken sowie ein zweiter, unabhängig von dem ersten Operationshaken zum Zurückziehen der Regenbogenhaut am Auge zu fixierender Operationshaken bekannt. Der zweite Operationshaken hat ein an beiden Enden mit einem umgebogenen Hakenteil versehenes gerades Teilstück, an welchem eine Scheibe aus flexiblem Material verstellbar angeordnet ist, wobei mittels der Scheibe ein zum Einführen des Operationshakens in der Hornhaut (Cornea) vorgesehener Einschnitt abgedichtet und somit ein Austritt des Augenwassers verhindert wird. Bei dem bekannten Operationshaken besteht das Problem, dass der einzelne, die Hornhaut durchdringende Einschnitt zum Einführen des jeweiligen Operationshakens relativ gross bemessen ist, so dass zur Vermeidung von unerwünschtem Austritt des Augenwassers einerseits der Einschnitt mit der flexiblen Scheibe ausreichend abgedichtet und nach dem Entfernen des Operationshakens durch zeitaufwendiges Vernähen wieder verschlossen werden muss.

Aus der EP-A 0 502 258 ist ein weiterer Operationshaken zur Verwendung beim Eingriff am Auge eines Lebewesens bekannt, welcher im wesentlichen ein Führungsteil mit angeformtem Hakenstück sowie ein am Führungsteil verschiebbares und als Plättchen ausgebildetes Klemmteil umfasst. Bei diesem Operationshaken ist das von dem Führungsteil durchdrungene Klemmteil mindestens auf der der äusseren Corneakontur zugewandten Seite mit einer kreisbogenförmigen Ausnehmung versehen, durch welche das Klemmteil etwa in zwei Teilstücke unterteilt und dadurch eine optimale Anpassung an die äussere Cornea-Kontur gewährleistet ist.

Der Erfindung liegt die Aufgabe zugrunde, einen Operationshaken dahingehend zu verbessern und so auszubilden, dass unter Beibehaltung einer möglichst präzisen Handhabung einerseits die Regenbogenhaut (Iris) beim Zurückziehen nicht verletzt wird, und dass andererseits der operationshaken ohne zusätzliche Abdichtung durch einen Einschnitt in der Hornhaut (Cornea) einführbar ist und ohne nachträgliches Vernähen oder dergleichen wieder entfernt werden kann.

Diese Aufgabe wird erfindungsgemäss dadurch gelöst, dass der kunststoffaden thermoplastisch ist und das Hakenelement mit dem angeformtem Einhängeteil im umgebogenen Bereich desselben eine lokal begrenzte Steifigkeit aufweist, welche von der Steifigkeit des restlichen kunststoffadens abweicht und geringer ist als der in eingehängtem Zustand durch die zurückgezogene Regenbogenhaut oder der in ausgehängtem Zustand durch die Hornhaut am Einhängeteil wirkende Widerstand, so dass beim Herausziehen des Operationshakens aus dem Auge der umgebogene Bereich des Einhängeteils durch den Widerstand in eine weitgehend gerade Stellung aufbiegbar ist.

Der erfindungsgemässe Operationshaken hat den Vorteil, dass zum Einführen in das Auge in der Hornhaut lediglich ein kleiner Einschnitt erforderlich ist, welcher so ausgebildet und bemessen ist, dass eine zusätzliche Abdichtfunktion durch das am Führungsteil verschiebbare Fixierelement nicht erforderlich ist.

Im Rahmen der Erfindung hat sich herausgestellt, dass das fadenförmige Führungsteil des Operationshakens während des operativen Eingriffs von den durch den Einschnitt getrennten Corneawänden selbstdichtend umschlossen wird. Die lokal begrenzte Steifigkeit am Einhängeteil hat zudem den Vorteil, dass zum Entfernen des Operationshakens bei entsprechender Zugbelastung am Führungsteil sich das hakenförmige Einhängeteil selbsttätig öffnet, so dass das Hakenelement im wesentlichen als länglicher Faden aus dem Auge herausgezogen werden kann. Weiterhin hat sich herausgestellt, dass nach dem Entfernen des Operationshakens sich der Einschnitt in der Cornea selbstabdichtend verschliesst, wodurch gleichzeitig ein unerwünschter Austritt der Augenflüssigkeit verhindert wird. Ein zusätzliches Vernähen des Einschnitts ist somit nicht mehr erforderlich. Das in bezug auf das Einhängeteil relativ lange, fadenförmige Führungsteil hat eine ausreichende Flexibilität, so dass durch ein unbeabsichtigtes Berühren durch den Operateur keine Verletzung am Auge verursacht wird.

Weitere Merkmale der Erfindung ergeben sich aus der Beschreibung in Verbindung mit der Zeichnung und den weiteren Patentansprüchen.

Die Erfindung wird nachstehend anhand der Zeichnung beschrieben. Es zeigt:
**Fig.1** ein in Seitenansicht und in grösserem Massstab dargestelltes Hakenelement für einen operationshaken;
**Fig.2** den in Seitenansicht dargestellten operationshaken, bestehend aus dem Hakenelement gemäss Fig.1 und einem daran angeordneten Fixierelement;
**Fig.3** den in Draufsicht dargestellten Operationshaken gemäss Pfeilrichtung III in Fig.2;
**Fig.4** ein schematisch, als Horizontalschnitt und in grösserem Massstab dargestelltes Teilstück eines Auges, bei welchem der eine Bereich der Regenbogenhaut (Iris) mit dem Operationshaken gemäss Fig.2 in zurückgezogener Stellung dargestellt ist;
**Fig.5** das in Draufsicht und in bezug auf Fig.4 kleiner dargestellte Auge mit teilweise zurückgezogen dargestellter Regenbogenhaut (Iris);
**Fig.6** ein Teilstück des Hakenelements für den Operationshaken gemäss Fig.2, bei welchem das hakenförmige Einhängeteil in weitgehend geöffnetem oder gestrecktem Zustand dargestellt ist; und
**Fig.7 bis 10** jeweils ein schematisch und in grösserem Massstab sowie als Horizontalschnitt dargestelltes Teilstück des Auges mit dem beim Herausziehen in verschiedenen Phasen dargestellten Operationshaken.

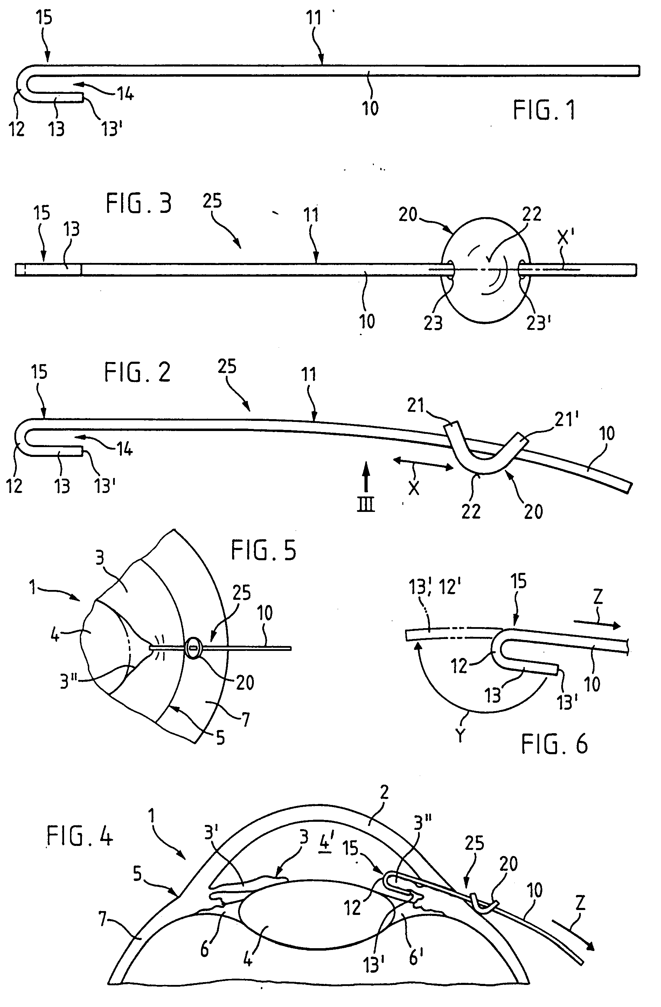

Fig.1 zeigt ein in Seitenansicht sowie in grösserem Massstab dargestelltes Hakenelement 10 für einen Operationshaken, mittels welchem beim operativen Eingriff am Auge die Regenbogenhaut (Iris) erfasst und nach aussen gezogen werden kann. Der Operationshaken (Iris-Retractor) umfasst, wie in Fig.2 dargestellt, im wesentlichen das Hakenelement 10 sowie ein verschiebbar und feststellbar daran angeordnetes Fixierelement 20.

Das Hakenelement 10 ist aus einem Faden mit absolut glatter Oberfläche hergestellt und umfasst, wie in Fig.1 dargestellt, im wesentlichen ein längliches Führungsteil 11 und ein hakenförmig ausgebildetes Einhängeteil 15, welches an dem einen Ende des Führungsteils 11 angeordnet ist. Das Einhängeteil 15 besteht aus einem Bogenteil 12 und einem im Abstand 14 zum Führungsteil 11 angeordneten Schenkel 13 mit rechtwinklig dazu angeordneter Stirnkante 13'. Die Stirnkante 13' ist in bezug auf den Schenkel 13 vorzugsweise als glatte, ebene Fläche ausgebildet. Das einmal haarnadelförmig umgebogene, am Führungsteil 11 angeformte Einhängeteil 15 ist beispielsweise derart ausgebildet, dass der Schenkel 13 sich parallel mit einem Abstand 14 zum Führungsteil 11 erstreckt.

Fig.2 zeigt den in der Gesamtheit dargestellten Operationshaken 25 und man erkennt das Hakenelement 10 gemäss Fig.1 mit dem daran angeordneten Fixierelement 20. Das beispielsweise als kreisförmige Scheibe ausgebildete und aus flexiblem Material hergestellte Fixierelement 20 ist, wie in Fig.3 dargestellt, mit zwei im Abstand zueinander angeordneten und das Fixierelement 20 durchdringende Bohrungen 23 und 23' versehen. Die beiden Bohrungen 23,23' sind derart ausgebildet und zueinander angeordnet, dass das Fixierelement 20 auf dem Führungsteil 11 des Hakenelements 10 in Doppelpfeilrichtung X verschoben werden kann. Hierbei wird das Fixierelement 20, wie in Fig.2 dargestellt, derart verformt, dass auf der einen Seite des Führungsteils 11 eine im Profilquerschnitt etwa bogenförmige Anlage 22 gebildet ist, während auf der anderen Seite des Führungsteils 11 zwei im Abstand zueinander angeordnete Teilstücke 21,21' hervorstehen. Zum Verschieben des Fixierelements 20 auf dem Führungsteil 11 werden die beiden Teilstücke 21,21' relativ zueinander zusammengedrückt, wodurch die nicht bezeichneten Innenkanten der Bohrungen 23,23' ausser Eingriff des Führungsteils 11 gelangen (nicht dargestellt). Sobald die beiden Teilstücke 21,21' losgelassen werden, wird das Fixierelement 20 selbsthemmend auf dem Hakenelement 10 fixiert.

In Fig.3 ist der Operationshaken 25 gemäss in Fig.2 eingezeichneter Pfeilrichtung III in Draufsicht dargestellt und man erkennt das aus dem Führungsteil 11 und dem Einhängeteil 15 gebildete Hakenelement 10 sowie das daran angeordnete Fixierelement 20. Die beiden beabstandeten und auf der gemeinsamen Symmetrieachse X' des Hakenelements 10 und des Fixierelements 20 angeordneten Bohrungen 23 und 23' sind vorzugsweise so ausgebildet und in bezug auf das fadenförmige Hakenelement 10 derart dimensioniert, dass das Fixierelement 20, wie bereits erwähnt, selbsthemmend auf dem Führungsteil 11 angeordnet und durch Zusammendrücken der beiden Teilstücke 21 und 21' in Doppelpfeilrichtung X (Fig. 2) auf dem Führungsteil 11 verstellbar ist. Durch Loslassen der beiden Teilstücke 21,21' wird das Fixierelement 20 selbsthemmend gehalten und kann somit in beliebiger Position auf dem Führungsteil 11 angeordnet werden.

An dieser Stelle wird darauf hingewiesen, dass zur Herstellung des Hakenelements 10 ein verhältnismässig flexibler Faden aus thermoplastischem Kunststoff, beispielsweise ein Polyamidfaden mit guten Oberflächen-Gleiteigenschaften verwendet wird. Zur Erreichung der in Fig.1 und Fig.2 dargestellten Formgebung des Hakenelements 10 wird der Faden durch eine geeignete Wärmebehandlung verformt.

Für die entsprechende Formgebung des Hakenelements 10 wird der Polyamidfaden auf einen dem Führungsteil 11 und dem Einhängeteil 15 entsprechend ausgebildeten Formkörper (nicht dargestellt) aufgebracht und zusammen mit dem Formkörper einer Wärmebehandlung (Temperung) unterzogen. Durch die Wärmebehandlung erhält der Polyamidfaden im Bereich des Einhängeteils 15 die hakenförmige Formgebung und dadurch eine lokale Steifigkeit. Das in bezug auf das Einhängeteil 15 relativ lange Führungsteil 11 bleibt nach der Wärmebehandlung weitgehend flexibel. Das Führungsteil 11 kann gerade (Fig.1) oder, wie in Fig.2 schematisch dargestellt, mit relativ grossem Radius bogenförmig ausgebildet werden.

Durch die vorstehend erwähnte Wärmebehandlung des Polyamidfadens wird eine derart lokale Steifigkeit des Einhängeteils 15 erreicht, dass bei entsprechendem Widerstand durch weiteren Zug (Pfeilrichtung Z) am Führungsteil 11 sich das hakenförmige Einhängeteil 15 durch Aufbiegen weitgehend in die ursprüngliche Lage zurückbewegt.

Das Fixierelement 20 ist aus flexiblem Kunststoff mit guten Gleiteigenschaften hergestellt, welches beim Zusammendrücken der beiden Teilstücke 21,21' weitgehend kraftlos auf dem Führungsteil 11 des Hakenelements 10 verschiebbar ist. Das Fixierelement 20 ist beispielsweise aus transparentem Silikon (Silikon-Kautschuk) hergestellt.

Fig.4 zeigt ein in der Gesamtheit mit 1 bezeichnetes Auge in schematisch dargestelltem Horizontalschnitt und man erkennt die Hornhaut 2 (Cornea), die Regenbogenhaut 3 (Iris) mit den Bereichen 3' und 3'', die Lederhaut 7 (Sklera), die Linse 4 (Okular), die Pupille 4' sowie die Strahlenbänder 6,6' (Zonula).

Zur Verdeutlichung der Erfindung ist in Fig.4 der mit dem Bogenstück 12 des Einhängeteils 15 an dem einen Bereich 3'' der Iris 3 eingehängte Operationshaken 25 dargestellt. Der Operationshaken 25 ist mittels der weitgehend am Übergang 5 anliegenden Anlage 22 des Fixierelements 20 gehalten. Das flexible und leicht bogenförmig ausgebildete Führungsteil 11 des Hakenelements 10 ist dabei etwa an die äussere Kontur der Lederhaut 7 (Sklera) angepasst. Hierdurch wird erreicht, dass auch bei mehreren, zirkulär im Abstand zueinander verteilt angeordneten Operationshaken 25 ein gut zugänglicher Operationsbereich gewährleistet ist und der Operateur beim Eingriff nicht am Führungsteil 11 des einzelnen Operationshakens 25 hängen bleibt.

Eine unbeabsichtigte Berührung des Führungsteils 11 durch den Operateur wird durch die Flexibilität des länglichen Führungsteils 11 kompensiert, so dass durch eine etwaige Berührung keine gefährliche Bewegung (Herausreissen des Operationshakens 25 oder Einwirkung auf die Linse 4) auf das Auge 1 übertragen wird.

Fig.5 zeigt ein in Draufsicht und in bezug auf Fig.4 etwas kleiner dargestelltes Teilstück des Auges 1 und man erkennt das teilweise mit dem Operationshaken 25 zurückgezogene Teilstück 3'' der Iris 3, wobei der Operationshaken 25 mit dem scheibenförmigen Fixierelement 20 am Übergang 5 gehalten ist.

In Fig.6 ist in grösserem Massstab das vordere, als Einhängeteil 15 ausgebildete Teilstück des Hakenelements 10 dargestellt. Durch die erwähnte Wärmebehandlung des Polyamidfadens wird im Bereich des Einhängeteils 15 eine lokale Steifigkeit erreicht. Die Steifigkeit ist jedoch nur so gross, dass mit dem Einhängeteil 15 in einer ersten Phase das Teilstück 3'' der Iris 3, wie in Fig.4 dargestellt, bis etwa zum äusseren Rand (Fig.5) zurückgezogen werden kann.

In einer zweiten Phase wird der Operationshaken 10 vorzugsweise entgegen der Pfeilrichtung Z derart nach innen geschoben, dass bei entspanntem Teilstück 3'' der Iris 3 (das Einhängeteil 15 ist ausser Eingriff der Iris 3) der Operationshaken 10 um etwa 70° bis 90° um seine Längsachse gedreht werden kann. Anschliessend wird der Operationshaken 10 in Pfeilrichtung Z gezogen. Sobald das hakenförmige Einhängeteil 15 mit der Stirnseite 13' des Schenkels 13 am inneren, nicht näher bezeichneten Übergang von der Hornhaut 2 (Cornea) zur Lederhaut 7 (Sklera) anliegt wird durch weiteren Zug in Pfeilrichtung Z das hakenförmige Einhängeteil 15 derart aufgebogen, dass der Operationshaken 10 als länglicher Faden herausgezogen werden kann. Sobald der Faden vollständig herausgezogen ist, erfolgt ein selbstabdichtendes Verschliessen des in der Hornhaut 2 vorgesehenen, nicht näher dargestellten Einschnittes.

Es besteht jedoch auch die Möglichkeit, dass ausgehend von der in Fig.4 dargestellten Stellung durch weiteres Ziehen am Führungsteil 11 in Pfeilrichtung Z der Operationshaken 10 entfernt werden kann. Hierbei wird durch die Steifigkeit des zurückgezogenen Teilstücks 3'' das Einhängeteil 15, wie in Fig.6 schematisch dargestellt, durch Aufbiegen etwa in die ursprüngliche, gestreckte Lage (gestrichelte Teile 12',13') bewegt und aus dem Auge herausgezogen, so dass auch hierbei unmittelbar nach dem Entfernen des Operationshakens 25 sich der Einschnitt in der Hornhaut 2 selbstabdichtend verschliesst.

Zur Verdeutlichung der Dimensionen des erfindungsgemässen Operationshakens 25 wird darauf hingewiesen, dass die gesamte Länge des Hakenelements 10 etwa in der Grössenordnung zwischen 5mm bis 8mm und die Länge des umgebogenen Einhängeteils 15 in der Grössenordnung zwischen 1,0mm bis 1,5mm liegt. Die gesamte Breite des Bogenteils 12 liegt in der Grössenordnung zwischen 0,4mm bis 0,5mm. Der Polyamidfaden ist beispielsweise im Profilquerschnitt kreisförmig ausgebildet und hat einen Durchmesser von etwa 0,15mm bis 0,2mm.

Durch die im umgebogenen Bereich des Einhängeteils begrenzte Steifigkeit des Einhängeteils 15, kann in eingehängtem und zurückgezogenem Zustand der Regenbogenhaut 3'' (Fig.4) sowie bei ausgehängtem Zustand (nicht dargestellt) des Einhängeteils 15 der erfindungsgemässe Operationshaken 25 mit weitgehend aufgebogenem Einhängeteil 15 problemlos aus dem Auge 1 herausgezogen werden, ohne dass der Einschnitt in der Hornhaut 2 (Cornea) durch Aufreissen unerwünscht vergrössert wird. Der Einschnitt in der Hornhaut 2 (Cornea) verschliesst sich nach dem Entfernen des Operationshakens 25 selbstabdichtend.

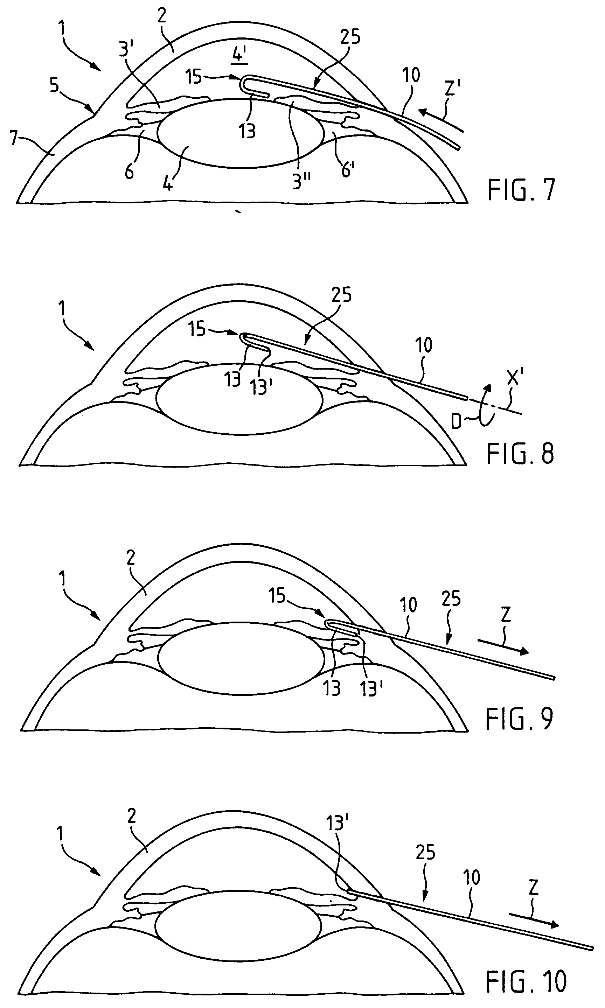

In den Figuren 7 bis 10 sind in grösserem Massstab und in verschiedenen Stellungen die wesentlichen Phasen des Operationshakens 25 beim Herausziehen aus dem Auge 1 dargestellt. Das nachstehend beschriebene Arbeitsverfahren zum Entfernen des Operationshakens 25 ist eine bevorzugte Variante des Verfahrens zum Entfernen des Operationshakens 25.

Fig.7 zeigt das Auge 1 im Horizontalschnitt analog Fig.4 und man erkennt die Hornhaut 2, die Regenbogenhaut 3 mit den beiden Bereichen 3' und 3'', die Lederhaut 7, die Linse 4, die Pupille 4' sowie die Strahlenbänder 6 und 6'. Abweichend von Fig.4 ist der Operationshaken 25 wie in Fig.7 dargestellt, soweit in Pfeilrichtung Z' geschoben, dass das Einhängeteil 15 mit dem Schenkel 13 ausser Eingriff des Teilstücks 3'' der Iris 3 ist.

In einer nächsten Phase wird der Operationshaken 25, wie in Fig.8 dargestellt, um seine Längsachse X' etwa um 70° bis 90° in Pfeilrichtung D gedreht. Anschliessend wird der Operationshaken 25, wie in Fig.9 dargestellt, in Pfeilrichtung Z gezogen.

Sobald das hakenförmige Einhängeteil 15, wie in Fig.9 dargestellt, mit der Stirnseite 13' des Schenkels 13 am inneren, nicht näher bezeichneten Übergang von der Hornhaut 2 (Cornea) zur Lederhaut 7 (Sklera) anliegt wird durch weiteren Zug in Pfeilrichtung Z das hakenförmige Einhängeteil 15 derart aufgebogen, dass der Operationshaken 10, wie in Fig.10 dargestellt, als länglicher und im wesentlichen gerader Faden aus dem Auge 1 herausgezogen werden kann.

## Patentansprüche

1. Operationshaken zur Verwendung beim chirurgischen Eingriff am Auge (1) eines Lebewesens, bestehend aus einem zum örtlichen Zurückziehen der Regenbogenhaut (3) an mindestens einem Ende mit einem angeformten hakenförmigen Einhängeteil (15) sowie mit einem länglichen Führungsteil (11) versehenen Hakenelement (10) aus einem flexiblen Kunststofffaden und einem an dem Führungsteil (11) verschiebbar angeordneten Fixierelement (20), mit welchem der durch einen Einschnitt in der Hornhaut (2) eingeführte Operationshaken (25) an der äusseren Kontur des Auges (1) haltbar ist, **dadurch gekennzeichnet**, dass der kunststoffaden thermoplastische ist und das Hakenelement (10) mit dem angeformten Einhängeteil (15) im umgebogenen Bereich (12) desselben eine lokal begrenzte Steifigkeit aufweist, welche von der Steifigkeit des festlichen Kunststoffadens abweicht und geringer ist als der in eingehängtem Zustand durch die zurückgezogene Regenbogenhaut (3'') oder der in ausgehängtem Zustand durch die Hornhaut (2) am Einhängeteil (15) wirkende Widerstand, so dass beim Herausziehen des Operationshakens (25) aus dem Auge (1) der umgebogene Bereich des Einhängeteils (15) durch den Widerstand in eine weitgehend gerade Stellung aufbiegbar ist.

2. Operationshaken nach Anspruch 1, **dadurch gekennzeichnet**, dass das hakenförmige Einhängeteil (15) durch eine thermische Behandlung am Hakenelement (10) angeformt ist.

3. Operationshaken nach Anspruch 1, **dadurch gekennzeichnet**, dass das mit dem angeformten Einhängeteil (15) versehene Hakenelement (10) aus einem Polyamidfaden hergestellt ist.

4. Operationshaken nach Anspruch 1, **dadurch gekennzeichnet**, dass das Fixierelement (20) als eine flexible Scheibe ausgebildet ist, welche in bezug auf ihre Symmetrieachse mindestens zwei im Abstand zueinander angeordnete Löcher (23,23') aufweist, durch welche das Führungsteil (11) des fadenförmigen Hakenelements (10) hindurchführbar ist.

5. Operationshaken nach Anspruch 4, dadurch gekennzeichnet, dass das Fixierelement (20) in aufgeschobenem Zustand auf das Hakenelement (10) eine Eigensteifigkeit aufweist, durch welche das in Längsrichtung am Führungsteil (11) verstellbare Fixierelement (20) in beliebiger Stellung selbsthemmend feststellbar ist.

6. Operationshaken nach den Ansprüchen 4 und 5, dadurch gekennzeichnet, dass das Fixierelement (20) derart ausgebildet ist, dass es in aufgeschobenem Zustand auf der einen Seite des Führungsteils (11) zwei beabstandete und zum Verstellen des Fixierelements (20) zusammendrückbare Teilstücke (21,21') und auf der anderen Seite eine bogenförmige Anlage (22) bildet, mit welcher Anlage (22) das Fixierelement (20) am Auge (1) gehalten und abgestützt ist.

7. Operationshaken nach Anspruch 4, dadurch gekennzeichnet, dass das Fixierelement (20) als eine von den beabstandeten, quer zur Oberfläche orientierten Löchern (23,23') durchdrungene Scheibe ausgebildet und aus flexiblem Material hergestellt ist, wobei das scheibenförmige Fixierelement (20) in seiner äusseren Formgebung kreisförmig, elliptisch, quadratisch oder rechteckig ausgebildet ist.

8. Operationshaken nach den Ansprüchen 4 bis 7, dadurch gekennzeichnet, dass das Fixierelement (20) aus Silikon-Kautschuk oder dergleichen hergestellt ist.

## Claims

1. Surgical hook for use during surgical treatment on the eye (1) of a living organism, comprising a hook element (10) made of a flexible plastic thread provided on at least one end with a moulded-on, hook-shaped engaging part (15) and with an elongated guide part (11) for localised retraction of the iris (3) and comprising a fixing element (20) displaceably arranged on the guide part (11), with which the surgical hook (25) inserted through an incision in the cornea (2) can be held against the outer contour of the eye (1), characterised in that the plastic thread is thermoplastic and the hook element (10) with the moulded-on engaging part (15) in the bent-over region (12) thereof has a locally restricted rigidity, which differs from the rigidity of the rest of the plastic thread and is less than the resistance acting on the engaging part (15) as a result of the retracted iris (3'') when engaged or as a result of the cornea (2) when disengaged, so that when the surgical hook (25) is drawn out from the eye (1), the bent-over region of the engaging part (15) may be bent upwards into a substantially straight position by the resistance.

2. Surgical hook according to Claim 1, characterised in that the hook-shaped engaging part (15) is moulded on the hook element (10) by a thermal treatment.

3. Surgical hook according to Claim 1, characterised in that the hook element (10) provided with the moulded-on engaging part (15) is made from a polyamide thread.

4. Surgical hook according to Claim 1, characterised in that the fixing element (20) is constructed as a flexible disc, which with respect to its axis of symmetry has at least two spaced holes (23, 23'), through which the guide part (11) of the thread-like hook element (10) may be passed.

5. Surgical hook according to Claim 4, characterised in that when pushed onto the hook element (10), the fixing element (20) has an inherent rigidity, as a result of which the fixing element (20) adjustable in the longitudinal direction of the guide part (11) may be fixed in any desired position in a self-locking manner.

6. Surgical hook according to Claims 4 and 5, characterised in that the fixing element (20) is constructed such that when pushed on, it forms two spaced part-sections (21, 21'), which may be compressed for adjustment of the fixing element (20), on one side of the guide part (11) and forms an arc-shaped abutment (22) on the other side, by means of which abutment (22) the fixing element (20) is held and supported against the eye (1).

7. Surgical hook according to Claim 4, characterised in that the fixing element (20) is constructed as a disc penetrated by the spaced holes (23, 23') oriented transversely to the surface and is made of flexible material, whereby the external shaping of the disc-shaped fixing element (20) is circular, elliptic, square or rectangular in construction.

8. Surgical hook according to Claims 4 to 7, characterised in that the fixing element (20) is made of silicon caoutchouc or the like.

## Revendications

1. Crochet à utiliser dans la chirurgie de l'oeil (1) d'un être vivant, composé d'un élément de crochet (10) réalisé dans un fil synthétique flexible, pourvu au moins sur une extrémité d'une portion d'accrochage (15) façonnée en forme de crochet et permettant une rétractation locale de l'iris (3) ainsi que d'une portion de guidage allongée (11), et d'un élément de fixation (20) disposé sur la portion de guidage (11) de façon mobile, grâce auquel élément de fixation le crochet chirurgical (25) introduit par une incision pratiquée dans la cornée (2) peut être retenu sur le contour externe de l'oeil (1), caractérisé en ce que le fil synthétique est thermoplastique et l'élément de crochet (10) avec la portion d'accrochage (15) façonnée présente dans une zone en arc (12) de celle-ci une raideur locale limitée, laquelle diffère de la raideur du reste du fil en matériau synthétique et est plus petite que la résistance agissant contre la portion d'accrochage (15), à l'état accroché par l'iris rétracté (3'') ou à l'état libéré par la cornée (2) de sorte que, lors du retrait du crochet chirurgical (25) de l'oeil (1), la zone en arc de la portion d'accrochage (15) peut être dépliée par la résistance pour avoir une position essentiellement droite.

2. Crochet chirurgical selon la revendication 1, caractérisé en ce que la portion d'accrochage (15) en forme de crochet est façonnée par un traitement thermique en un élément de crochet (10).

3. Crochet chirurgical selon la revendication 1, caractérisé en ce que l'élément de crochet (10) équipé de la portion d'accrochage (15) façonnée est fabriqué à partir d'un fil de polyamide.

4. Crochet chirurgical selon la revendication 1, caractérisé en ce que l'élément de fixation (20) est conçu en tant que disque flexible, lequel présente, par rapport à son axe de symétrie, au moins deux orifices (23, 23') disposés en formant un écart, par lesquels la portion de guidage (11) de l'élément de crochet (10) en forme de fil peut être introduite.

5. Crochet chirurgical selon la revendication 4, caractérisé en ce que l'élément de fixation (20) présente, à l'état poussé, sur l'élément de crochet (10) une raideur propre, par le biais de laquelle l'élément de fixation (20) pouvant être déplacé sur la portion de guidage (11) dans la direction longitudinale, peut être immobilisé dans une position quelconque de façon auto-bloquante.

6. Crochet chirurgical selon les revendications 4 et 5, caractérisé en ce que l'élément de fixation (20) est conçu de façon telle qu'il forme, à l'état poussé, sur un côté de la portion de guidage (11), deux pièces (21, 21') disposées avec un écart et pouvant être comprimées pour régler l'élément de fixation (20), et sur l'autre côté, un dispositif en arc (22), avec lequel dispositif (22) l'élément de fixation (20) est maintenu et repose sur l'oeil (1).

7. Crochet chirurgical selon la revendication 4, caractérisé en ce que l'élément de fixation (20) est conçu en tant que disque traversé par les trous (23, 23') disposés selon un écart et orientés perpendiculairement à la surface, et est fabriqué dans un matériau flexible, l'élément de fixation (20) en forme de disque étant conçu, dans son façonnage externe, sous la forme d'un cercle, d'une ellipse, d'un quadrilatère ou d'un rectangle.

8. Crochet chirurgical selon les revendications 4 à 7, caractérisé en ce que l'élément de fixation (20) est fabriqué dans du caoutchouc siliconé ou équivalent.
